# EUROPEAN PATENT APPLICATION

(11) **EP 1 258 241 A1**
(43) Date of publication of application: **20.11.2002**
(21) Application number: 01830310.7
(22) Date of filing: 16.05.2001
(51) Int. Cl.: A61K 9/14, A61K 9/22

(54) **Method of increasing the bioavailability of nimesulide**

(71) Applicant: BIOPROGRESS S.p.A., I-00165 Rome (IT)
(72) Inventor: De Angelis, Pasquale, 03100 Frosinone (IT); Olivieri, Aldo, Roma (IT)
(74) Representative: Tansini, Elio Fabrizio

(57) **Abstract**

The present invention relates to a method of making nimesulide more bioavailable by adsorption on a polymeric carrier, with use of the solubilization technique in an organic solvent or by co-griding in a high-energy mill, with formation of an inclusion complex [nimesulide:polymeric carrier] of improved bio-pharmaceutical qualities. In addition, the present invention relates to physiologically acceptable pharmaceutical compositions containing the [nimesulide:polymeric carrier] complex as well as to the relevant use of same.

## Description

The present invention relates to a method of making nimesulide more bioavailable by adsorption on a polymeric carrier, through use of the solubilization technique in an organic solvent or, by co-grinding in a high-energy mill, with formation of an inclusion complex [nimesulide: polymeric carrier] of improved biopharmaceutical qualities.

In addition the present invention relates to physiologically acceptable pharmaceutical compositions containing the inclusion complex [nimesulide: polymeric carrier] as well as to the related use of same.

Nimesulide [N-(4-nitro-2-phenoxyphenyl)methane-sulfoanilide] is a molecule having an anti-inflammatory, antipyretic and analgesic action which belongs to the group of non-steroidal anti-inflammatory drugs.

Nimesulide is a weakly acid molecule (pKa = 6.5) and differs from other non-steroidal anti-inflammatory drugs because its acid group is a sulfoanilide and not a classic acidic group which is typical of the last-mentioned family of molecules. The chemical structure of nimesulide explains its very low solubility in water and in polar solvents.

Nimesulide is a drug above all employed in the therapy of painful articular diseases having a chronic course. Above all in case of prolonged use, nimesulide shares the tolerability profile of other similar molecules, characterized by specific injuring capacity to the detriment of the digestive system, with appearance of ulcers, bleeding and perforations.

Nimesulide is weakly soluble in water and in polar solvents.

Nimesulide is insoluble in diluted acid solutions, fairly soluble in chloroform, soluble in acetone, methylene chloride and solutions of 1N sodium hydroxide, weakly soluble in methyl alcohol and ethanol.

The solubility profile of nimesulide explains why there are mainly oral solid pharmaceutical forms (tablets, capsules and packets of granules for extemporary suspensions) and rectal forms (suppositories).

The pharmacological properties highlight that it exerts a weak activity inhibiting synthesis of prostaglandins "in vitro".

"In vivo", with pharmacologically active doses, it does not inhibit the cyclo-oxygenase enzyme at the gastric wall, whereas it stops prostaglandins production where inflammation occurs (direct action on 2 cyclo-oxygenase, COX2).

Furthermore, it was recently disclosed a mechanism of the scavenger type through which nimesulide restricts formation of superoxide radicals produced during the cascade of the arachidonic acid and released during the phlogistic process by leucocytes.

By analysing the pharmacokinetics of the preparations presently available on the market, one can become aware of the fact that oral administration has a maximum peak of haematic concentration (Cmax) around the second-third hour, with a half time included between 2 and 5 hours, showing efficient haematic concentrations until the sixth-eight hour.

The rectal administration on the contrary has a Cmax around the fourth hour, with a half time of about 5 hours.

Excretion mainly takes place through the urinary tract without giving rise to accumulation phenomena, even after repeated administrations.

Nimesulide, as almost all non-steroidal anti-inflammatory drugs, is therapeutically used in non-painful phlogistic states even when accompanied by pyrexia, in particular concerning the osteoarticulation system.

One of the greatest drawbacks in use of nimesulide for painful syndromes is due to the fact that the Cmax is relatively delayed as it appears between 1.5 and 4 hours [Bernareggi A., Drugs, 46 suppl. 1, pages 64-72, (1993)].

Values related to Cmax are very important above all in acute painful states, where nimesulide is required to have a high swiftness of action and where nimesulide is required to perform its action in a short period of time after administration, within an hour for example.

Swiftness of action is directly connected with "bioavailability" of the nimesulide "in vivo" and, therefore, with the capability of same to reach the biologic fluids more quickly, which from a merely physico-chemical point of view results in a greater dissolution velocity of nimesulide in water.

In addition, pharmaceutical formulations containing controlled-release nimesulide are desirable for the purpose of obtaining lower concentrations of the active ingredient in the serum and tissue, but more prolonged in time.

The controlled-release formulations would enable the same therapeutical effect to be achieved while reducing the number of daily administrations, reducing the daily dose of the active ingredient, and reaching efficient haematic and tissular levels of the active ingredient.

Therefore, there is still a need for pharmaceutical forms of nimesulide capable of overcoming the drawbacks present in the pharmaceutical forms that are already commercially available.

The technical problem that the present invention wishes to solve is to overcome the limits and drawbacks of the known art.

In particular, the technical problem that the present invention wishes to solve is to modify the absorption kinetics of the nimesulide active ingredient "in vivo".

The solution to the above mentioned technical problem is offered by the Applicant of the present invention that has surprisingly found that by changing the original structure (in the following "destructuration") of nimesulide, the same acquires a greater capability of being formed into a solution.

The Applicant has found that destructuration of the nimesulide involves transformation of its original crystalline structure to an amorphous form which has a greater tendency to become a solution as compared with the crystalline form.

The Applicant has selected a group of substrates, carriers of the polymeric type in order to obtain an inclusion complex [nimesulide: polymeric carrier] containing nimesulide in an amorphous form which has a greater bioavailability than nimesulide in a crystalline form.

The complex [nimesulide: polymeric carrier] enables therapeutical concentrations of the nimesulide active ingredient in the serum and therefore in the tissue with a maximum peak of action within the first hour of administration.

In addition, the complex [nimesulide: polymeric carrier] shows a therapeutical action over a period of time greatly higher than the period of time that can be obtained using the oral formulations presently available on the market.

It is an object of the present invention to provide an inclusion complex [nimesulide: polymeric carrier] having the features set forth in the accompanying independent claim.

It is another object of the present invention to provide a pharmaceutically acceptable solid pharmaceutical form having the features set forth in the accompanying independent claim.

It is a further object of the present invention to provide use of a solid pharmaceutical form as a medicament, as set forth in the accompanying independent claim.

Preferred embodiments are set forth in the accompanying dependent claims.

Further features and advantages of the invention will become more apparent from the description of a preferred embodiment given by way of non-limiting example with reference to the accompanying drawings, in which:
- Fig. 1 shows a thermal analysis spectrum (DSC) carried out on a sample [Nimesulide: Crospovidone = 1:4 w/w] prepared in accordance with example 2;
- Fig. 2 shows an X-ray diffraction spectrum carried out on nimesulide;
- Fig. 3 shows an X-ray diffraction spectrum carried out on nimesulide dissolved in acetone and dry-concentrated;
- Fig. 4 shows an X-ray diffraction spectrum carried out on Crospovidone polymeric carrier;
- Fig. 5 shows an X-ray diffraction spectrum of a sample [nimesulide: polymeric carrier];
- Fig. 6 shows an FTIR spectrum carried out on nimesulide;
- Fig. 7 shows an FTIR spectrum carried out on the NSP [Nimesulide Polymeric Carrier] complex prepared in accordance with example 1;
- Fig. 8 shows an X-ray diffraction spectrum of a direct mixture (nimes-ulide/polymeric carrier) after treatment with a solvent.

In the inclusion complex by adsorption [nimesulide:polymeric carrier] being the object of the present invention the nimesulide active ingredient [N-(4-nitro-2-phenoxyphenyl)methanesulfoanilide] does not show a crystalline structure.

The complex [nimesulide:polymeric carrier] is in a solid form and nimesulide is adsorbed on the polymeric carrier.

In said complex nimesulide does not show an ordered (crystalline) structure but it is present in an "amorphous" form.

Nimesulide in an amorphous form has better passage features into a solution than nimesulide in a crystalline form.

The polymeric carrier is a pharmaceutically acceptable polymer selected from the group consisting of hydrophilic polymers insoluble in water.

In the complex [nimesulide:polymeric carrier] the nimesulide and polymeric carrier are in a ratio by weight included between 1:10 and 10:1 w/w.

Advantageously, the hydrophilic polymer insoluble in water is cross-linked polyvinyl pyrrolidone and derivatives thereof.

Other polymers belonging to the class of cross-linked polyvinyl pyrrolidone are Polyplasdone XL, Polyplasdone XL-1, Polyplasdone INF-10 differing from Crospovidone due to their particle size.

Advantageously, to be employed as coating are water-soluble polymers selected from Eudragit 12.5 P, Eudragit S 12.5 P and polyethylene glycol 400 and water-dispersible polymers selected from the group comprising:
polyethylene glycol of a molecular weight included between 6000 and 24,000 (PEG), celluloses, hydroxypropyl cellulose (HPC), Eudragit S 12.5 and Eudragit RS 12.5.

Preferably, the nimesulide and polymeric carrier are in a ratio by weight included between 1:5 and 5:1; more preferably in a ratio by weight included between 1:4 and 4:1.

The process for preparing the inclusion complex [nimesulide:polymeric carrier] involves a step in which nimesulide is adsorbed on a polymeric carrier of the above described type.

Nimesulide is adsorbed on a hydrophilic carrier which is insoluble in water, by use of an appropriate solvent or, alternatively, by mechanical activation to obtain a complex [nimesulide:polymeric carrier] in a solid form containing the nimesulide active ingredient in an amorphous form.

In a preferred embodiment, the step of adsorbing nimesulide on a polymeric carrier takes place by:
- preparing a solution comprising nimesulide and a polar organic solvent;
- adding a predetermined portion of the solution comprising the nimesulide and a predetermined portion of polymeric carrier;
- eliminating the solvent employed in the solution until an active ingredient having a general formula [Nimesulide:polymeric carrier] is obtained in which the nimesulide is present in an amorphous form.

Practically, it happens that the nimesulide taken separately is a molecule insoluble in water and the polymeric carrier taken separately is a hydrophilic polymer insoluble in water.

Under conditions adapted to promote adsorption of the nimesulide on the polymer carrier an inclusion complex [nimesulide:polymeric carrier] is formed.

Alternatively, in another preferred embodiment, the step of adsorbing the nimesulide on a polymeric carrier takes place by:
- pre-mixing a predetermined portion of powdered nimesulide with a predetermined portion of powdered polymeric carrier until a homogeneous nimesulide and polymeric carrier mixture is obtained;
- co-grinding the nimesulide and polymeric carrier mixture in a high-energy mill until an active ingredient is obtained which has a general formula [Nimesulide:polymeric carrier] in which the nimesulide is present in an amorphous form.

Optimization of the process for obtaining the complex [Nimesulide:polymeric carrier] was carried out by correlating the technological preparation parameters with some features of the preparation, such as:
a) degree of destructuration : index of the activation degree achieved by the active ingredient;
b) solubilization kinetics: index of the thermodynamic activity reached by the active ingredient;
c) dissolution velocity: it enables evaluation of both the thermodynamic parameters and other physico-chemical features such as wettability and particle size.

By virtue of the particular thermodynamic state of the activated form, the dissolution velocity of nimesulide in the inclusion complex [nimesulide:polymeric carrier] is higher than that of the nimesulide active ingredient just as it is.

The best biopharmaceutical features bringing to a higher bioavailability of the active ingredient were checked by tests carried out "in vitro" by comparing the dissolution capability of nimesulide adsorbed on a polymeric carrier with a nimesulide just as it is, in normal simulated biological fluids (buffers, for example).

The above gives rise, from a pharmacokinetic point of view to achievement of the Cmax in quicker periods of time as compared with presently available oral forms (1.4-4 hours) and, from a clinical point of view, to a quicker arising of the analgesic effect.

This result would enable the dosage, presently of 100-200 mg/dose, to be reduced so that less medicament is advantageously employed for a patient. The greater quickness of action and the consequent welfare effect would be accompanied by a lower incidence of side effects.

The improved pharmacodynamic and pharmacokinetic properties make use of nimesulide more appropriate even in phlogistic states with a marked painful component.

As regards formation of the inclusion complex, a method was identified which contemplates dissolution of the active ingredient in an appropriate solvent and subsequent adsorption of same on the polymeric carrier, as well as a method of adsorption carried out by co-grinding.

Advantageously as a polymeric carrier, hydrophilic polymers insoluble in water were used such as cross-linked polyvinylpyrrolidone (PVP - homopolymer of the 2-vinylpyrrolidone) (commercial name: Crospovidone), obtained by a particular polymerisation technique called "pop-corn" technique giving the structure a high adsorption capacity towards organic molecules and, at the same time, a high capacity to release them.

Advantageously, Crospovidone was employed.

The invention further contemplates use of water-soluble and/or water-dispersible polymers such as polyethylene glycol (PEG) having a high (6000-24,000) molecular weight, hydroxypropylcellulose (HPC) and other chemically compatible polymers.

It should be noted that these polymers are used in common pharmaceutical practice and their tolerability by the organism has been known for long.

The required analytical tests to highlight the occurred formation of the complex [nimesulide: polymeric carrier] called Nimesulide Polymeric Carrier NSP were carried out, as well as studies relating to the dissolution kinetics with respect to nimesulide just as it is.

The classic analytical methods utilised confirmed the occurred adsorption of the active ingredient on the selected polymeric carrier, through interactions of the electrostatic type, as well as the possibility of highlighting the reactivity of the biochemical type, "in vitro", towards cyclo-oxygenase 2, COX2.

These methods are: differential scan calorimetry (DSC), X-ray diffractometry and FTIR spectroscopy.

It is a further object of the present invention to provide a pharmaceutically acceptable solid formulation comprising the complex [nimesulide:polymeric carrier] for use as a medicament. The solid pharmaceutical forms being the object of the present invention can be oral solid pharmaceutical forms (tablets, capsules and packets of granules for extemporary suspensions) and rectal forms (suppositories).

The above mentioned formulations involve:
a) immediate release of part of the active ingredient in contact with the gastric juice;
b) protection of a "first" part of the NSP until it reaches the first portion of the small intestine, where by virtue of its improved biopharmaceutical features, nimesulide is quickly released and quickly absorbed in the biological fluids;
c) protection of a "second" part of the NSP until the large intestine.

The pharmaceutical formulation being the object of the present invention also involves use, in association with the gastro-resistant and coating material, of one or more of all surface-active agents that are acceptable from a pharmaceutical point of view, for the purpose of facilitating and further increasing absorption of Nimesulide and improving bioavailability of same.

Preferably, surface-active agents are selected from:
1. Non-ionic surface-active agents, selected from: polyoxyalkylene ethers of higher alcohols, in particular those corresponding to the general formula: RO-[(CH₂)ₙ-O)]ₓ-H, where R can represent a higher alkane, an alkylphenol or a stearoil residue;
2. Ionic surface-active agents selected from: pharmaceutically acceptable quaternary ammonium salt, betaines, alkylbetaines, alkylamidopropylbetaines; benzalkonium chloride, dodecyl sodium sulfate; and
3. Natural surface-active agents selected from: lecithins from soybean and eggs for example, that are very efficient in promoting absorption of the active ingredient.

In accordance with the invention, surface-active agents are contained in a ratio varying from 0.01 to 10 parts of surface-active agent for 100 parts of nimesulide.

The compositions of the invention, as already said before, comprise use of a gastro-resistant and entero-soluble material, so as to enable part of the active ingredient to pass through the stomach in an intact state (a quite acid pH condition) and a certain amount thereof to be released in the small intestine (pH < 7) and a final amount thereof to be released in the large intestine (pH > 7).

Examples of substances adapted for gastric protection are: cellulose acetophthalate, Eudragit® L and S; a mixture consisting of 38% polymetacrylic acid - 46.7% polyvinylacetate - 7.6% stearic acid - 7.7% tetrahydrofuryl oleate - lac, and all other known substances giving the pharmaceutical active ingredients gastro-resistance and entero-solubility.

The formulation being the object of the present invention can be accomplished in the form of tablets or gastro-protected pills, gastro-protected microgranules with or without an inert material of the sugar type as a medium and formulated in capsules or packets, gastro-protected particles of NSP in an aqueous suspension which may be ready or extemporaneous, in a single or multi-dose form.

The compositions of the present invention contain NSP amounts capable of constituting unitary doses (single administration amount) of the active ingredient included between 10 and 500 mg.

The following examples further illustrate the invention without limiting it at all.

### The material employed in the experimental tests are:

Nimesulide commercially available from ERREGIERRE (Italy) A batch of cross-linked polyvinyl pirrolydone (Crospovidone) was supplied from Eigenmann & Veronelli (Italy).

Preparation examples of the activated system [nimesulide:polymeric carrier] are reproduced hereinafter.

### EXAMPLE 1 (Activation by use of a solvent)

The activated system [nimesulide: polymeric carrier = 1:4 w/w] was obtained by for example treating 10 g of polymer with 2.5 g nimesulide solution in 5 ml acetone.

Subsequently the solvent was removed under vacuum at a controlled temperature and the powder thus obtained was sieved (60 mesh) and homogenized.

### EXAMPLE 2 (Activation by co-grinding in a "high-energy" mill

The activated system [nimesulide:polymeric carrier = 1:4, w/w] was obtained by treating the powered nimesulide and polymeric carrier in a pre-mixing step over a period of time of 30 minutes.

The obtained mixture was subsequently submitted to a co-grinding step in a high-energy mill over a period of time of 3 hours, under controlled experimental conditions (phase displacement angle of 30°).

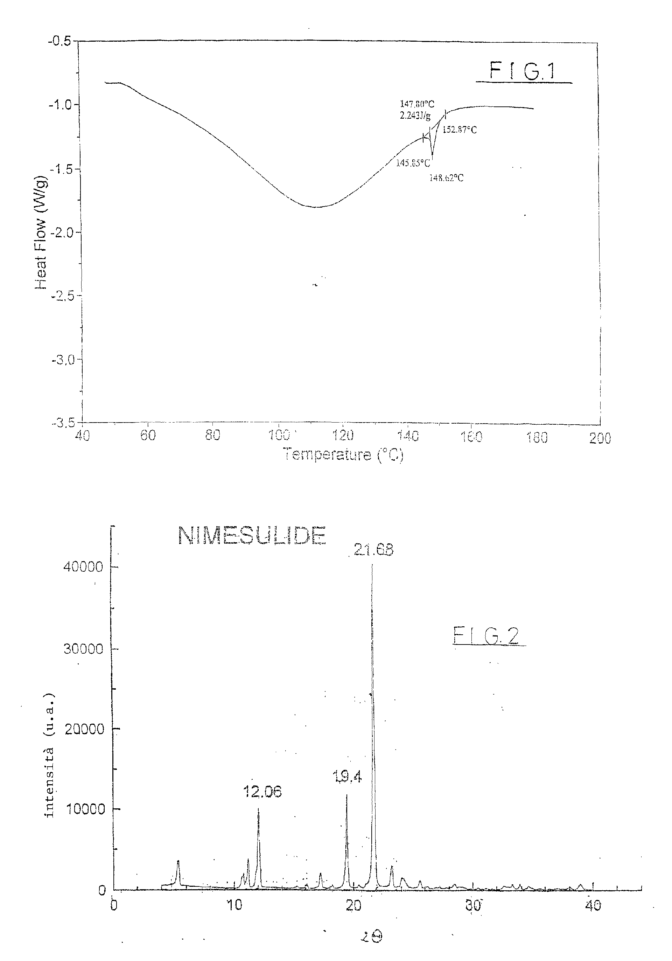

The powder thus obtained was sieved (60 mesh) and submitted to the provided analytical controls. The thermogram relating to a sample co-ground for 3 hours with a phase displacement angle of 30° highlights a percentage of residual crystallinity of 7-9% (ΔHf about 2.4 J/g), FIGURE 1. This residual-crystallinity value is a good result because values lower than 10% are considered as acceptable results.

The Applicant has carried out analytical controls on samples of the starting products, control samples and the activated system.

### Thermal Analysis

Thermal analysis was conducted by Differential Scan Calorimetry (DSC); analyses were carried out in a nitrogen flux (50 ml/min-1) at a heating speed of 10°C min-1.

### X-ray Diffractometric Analysis

The analysis was carried out on powdered samples (2θ:5° ÷ 40°).

### FTIR Analysis

Samples were analysed in nujol.

The thermal analysis (DSC) conducted on nimesulide shows a solid/liquid transition endotherm alone in the gap between 146.35°C and 164.10°C with a peak T° corresponding to 152.11°C, with an enthalpy of 156.5 J/g.

The thermogram was also recorded against time (in minutes), always heating with an increase of 10°C/minute; then the cooling step (10°C/minute) was carried out followed by a new heating step still with the same thermal increase.

Such a thermogram, taken as a whole, shows a solid/liquid transition endotherm (between 10' and 11'). In the subsequent cooling step no peak to be ascribed to recrystallization of nimesulide is noticed.

The X-ray diffraction spectrum shows a highly crystalline structure, FIGURE 2.

Also recorded was the thermogram relating to nimesulide dissolved in acetone and subsequently dry-concentrated. This further investigation was made necessary due to the use of this solvent in the preparation process for the activated form and, more specifically, in the nimesulide adsorption step on the cross-linked polymer. It is to be noted that nimesulide, after elimination under vacuum of the solvent, is reorganized into two crystalline structures (two very close solid-liquid transition peaks).

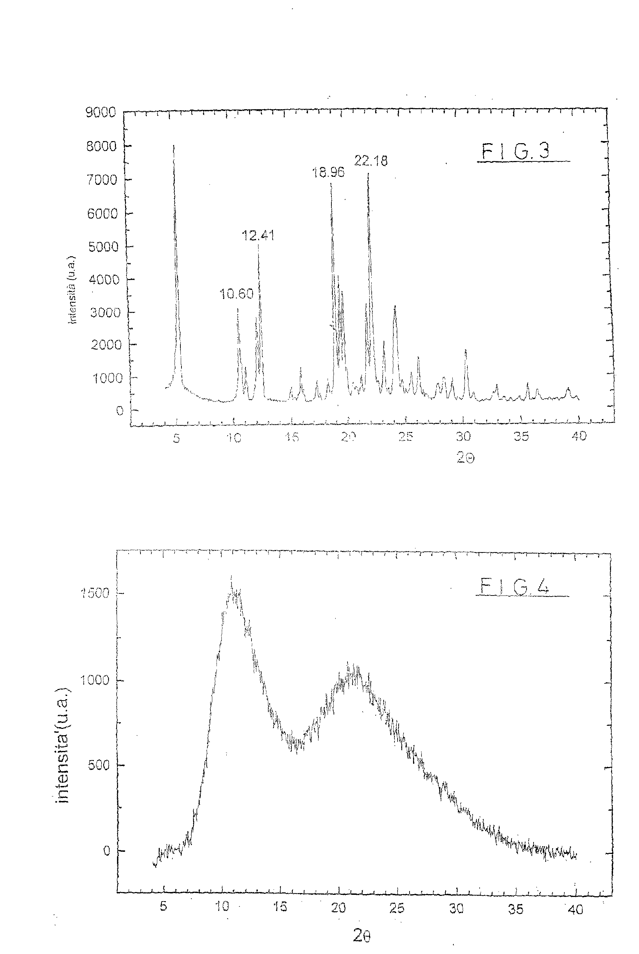

In this context, a comparison between the X-ray diffraction spectra of the nimesulide just as it is FIGURE 2 and nimesulide dissolved in acetone and dry-concentrated, does not show any amorphous character of the active ingredient, FIGURE 3.

The thermal analysis by DSC of Crospovidone shows a greatly widened endothermic curve included between about 62 and 178°C due to loss of the water contained in the polymer.

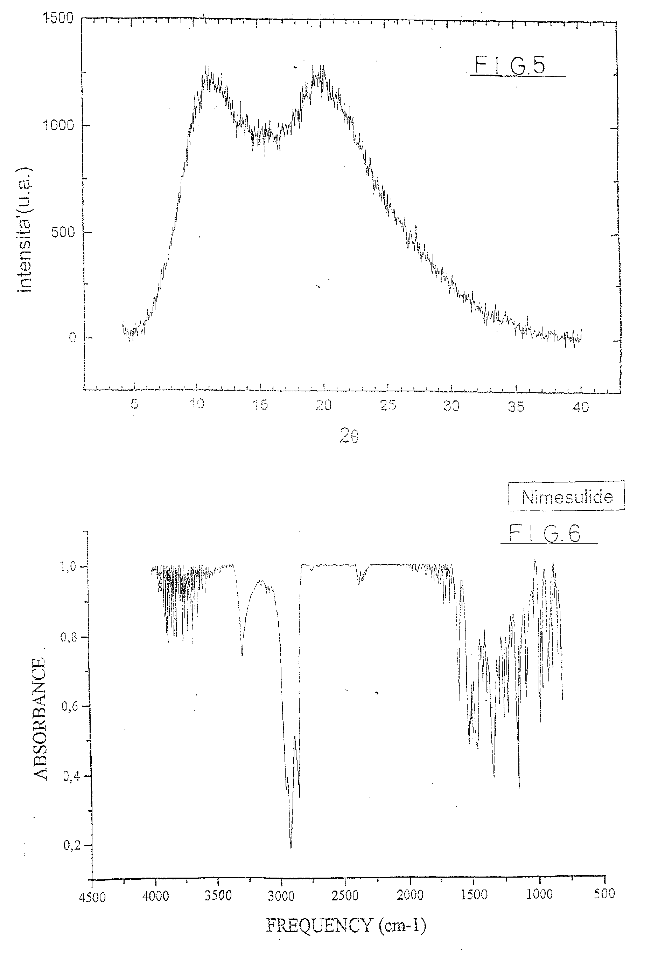

The X-ray diffraction analysis is typical of an amorphous substance, FIGURE 4.

The DSC thermal analysis carried out on a sample of NSP (activated complex NSP) prepared following Example 1, shows a greatly widened endothermic curve, included between 61 and 148°C, but the melting peak of nimesulide is not reached.

The X-ray diffraction spectrum confirms the presence of nimesulide in an "amorphous" state, FIGURE 5.

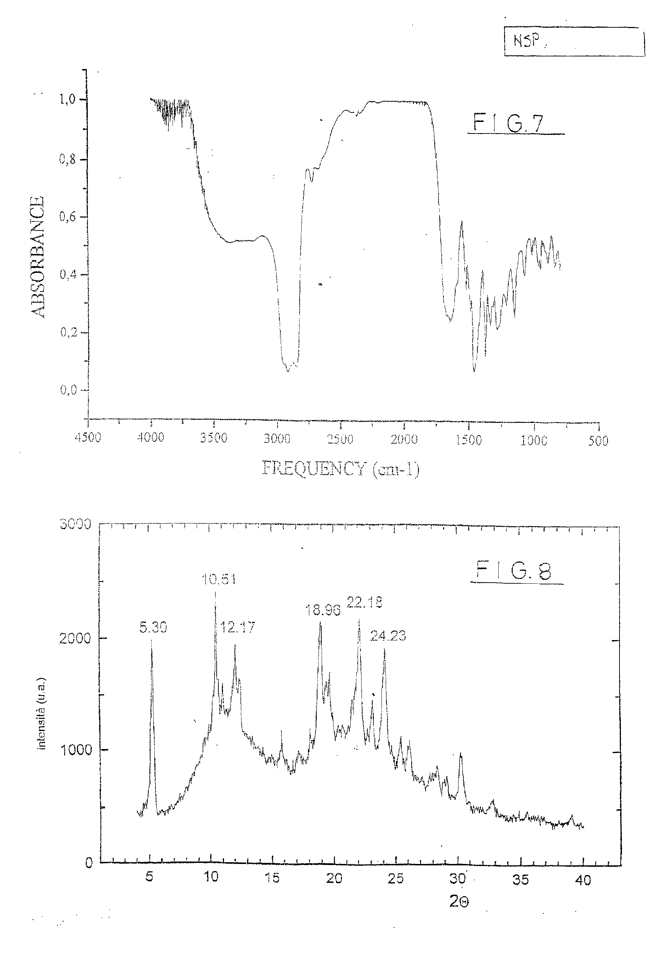

A comparison between the FTIR spectra, carried out on the nimesulide just as it is, FIGURE 6 and on the NSP, FIGURE 7, shows the disappearance in the latter of the signal relating to NH, to be most likely ascribed to electrostatic interactions between the active ingredient and polymeric medium, as a confirmation of the occurred adsorption.

Mixtures prepared in accordance with Example 1, with the following ratios (w/w) [nimesulide: polymeric carrier (PVP)]:

| | | | | |
|---|---|---|---|---|
| 0.5:1 | 1:1 | 1.5:1 | 2:1 | 2.12:1 |

were analysed in DSC. The related thermograms show that a partial adsorption of the active ingredient is obtained.

Advantageously, the nimesulide/polymeric carrier ratio 1:4 w/w has shown a greater percentage of the adsorbed active ingredient.

For the purpose of definitively excluding influence of the solvent utilised in preparing the activated complex, nimesulide and cross-linked PVP were treated separately in acetone; after solvent removal, the two solids were mixed (nimesulide : PVP ratio = 1:4 w/w) and the mixture was analysed. The related thermogram shows the presence of the two crystalline forms in nimesulide, highlighted by the X-ray diffraction spectrum, FIGURE 6.

The presence in the FTIR spectrum of the band relating to NH (3300 cm-1) shows the absence of electrostatic interactions between nimesulide and Crospovidone when a direct mixture (a mere mechanical mixing) is carried out between the two powders previously treated with acetone.

Given hereinafter are examples of controlled-release pharmaceutical formulations being the object of the present invention.

FORMULATION 1: repeated-action granules with quick breakdown or disgregation (stomach), at pH > 6 (small intestine) and at pH > 7 (larae intestine).

| **Description** | **Components** | | **Technology therein employed** |
|---|---|---|---|
| Coating A: protective film soluble at a pH < 5 | NSP | 500 mg | Microgranules are obtained by coating of NSP in Glatt (fluidized bed), until a constant weight and full and homogeneous uniformity |
| | (equal to nimesulide | 100 mg) | |
| | Hydroxyethylcellulose | 60 mg | |
| | | | |
| | Triethyl citrate | 5 mg | |
| | Talc | 1mg | |
| Coating B: protective film with mixed lattice with portion soluble at pH > 6.00 and insoluble part | NSP | 250 mg | Microgranules are obtained by coating of NSP in Glatt (fluidized bed), until a constant weight and full and homogeneous uniformity |
| | (equal to nimesulide | 50 mg) | |
| | Eudragit L 12.5 (dry substance) | (32 mg) | |
| | Eudragit S 12.5 (dry substance) | (15 mg) | |
| | PEG 400 | 37 mg | |
| | Triethyl citrate | 3 mg | |
| | Soybean lecithin | 2 mg | |
| | Talc | 1 mg | |
| Coating C: protective film with mixed lattice with portion soluble at pH > 7.00 and insoluble part | NSP | 250 mg | Microngranules are obtained by coating of NSP in Glatt (fluidized bed), until a constant weight and full and homogeneous uniformity |
| | (equal to nimesulide | 50 mg) | |
| | Eudragit S 12.5 P (dry substance) | (32 mg) | |
| | Eudragit RS (15 mg) (dry substance) | 12.5 | |
| | PEG 400 | 50 mg | |
| | Soybean lecithin | 2mg | |
| | Triethyl citrate | 3 mg | |
| | Talc | 1 mg | |
| Mixing of microgranules with coating A -B - C | | | Mixing of microgranules obtained in Glatt is carried out in a normal biconic mixer, over a period of time at least as long as 20' |
| Preparation of microgranules excipients and vehicles | C.M.C. Na | 250 mg | Components are sieved and submitted to a dry-granulation process, with formation of microgranules of the same size as the mixture of microgranules A -B and C |
| | Polyvinyl pyrrolidone | 250 mg | |
| | Sorbitol powder | 1000 mg | |
| | Aspartame | 15 mg | |
| | Precipitated silica | 120 mg | |
| | Fruit flavour powder | 55 mg | |
| Final mixing | Mixing of microgranules A - B and C + microgranules excipients and vehicles | | The final mixing of microgranules is carried out in a normal biconic mixer over a period of time at least as long as 30' |
| Envelope of coupled Al/Paper/PE | Heatsealed packets of appropriate sizes and measure | | The mixture is automatically packaged at the described weight by an appropriate packaging machine with final sealing. |

### STUDY ON THE CONTROLLED RELEASE "IN VITRO" OF FORMULATION 1

The microgranule mixture was submitted to control of dissolution "in vitro" by the USP XXIII method modified in the composition of the simulated gastrointestinal fluids (of an exactly known volume).

The amount of nimesulide was determined by HPLC, against an external standard of known title: analyses gave the following results:
A. After 1 hour (pH 1.50): the dosed-nimesulide amount is equal to 97.90 mg (first simulating liquid);
B. From the second to the third hour (pH 4.5 ÷ 6.9): the dosed-nimesulide amount is equal to 3.95 mg (second simulating liquid);
C. After 4 hours (pH 6.90): the dosed-nimesulide amount is equal to 47.21 mg (third simulating liquid):
D. From the fifth to the seventh hour (pH 6.90 ÷ 7.20): the dosed-nimesulide amount is equal to 3.10 mg (third simulating liquid);
E. After 8 hours (pH 7.20): the dosed-nimesulide amount is equal to 49.05 mg (fourth simulating liquid).

FORMULATION 2: repeated-action tablets with quick breakdown (stomach), at pH > 6 (small intestine) and at pH > 7 (large intestine).

| **Description** | **Components** | | **Technology therein employed** |
|---|---|---|---|
| Microgranules A: Preparation of compacted nimesulide with formation of granules of controlled size | NSP | 500 mg | Microgranules of controlled particle size are obtained by compacting the dry mixture, by means of a Ronchi compacting machine, and subsequent passage on a granulator with a welldefined mesh size |
| | (equal to nimesulide | 100 mg) | |
| | Magnesium stearate | 2.5 mg | |
| Coating B: protective film with mixed lattice with portion soluble at pH > 6.00 + insoluble part | NSP | 250 mg | Microgranules are obtained by coating of NSP in Glatt (fluidized bed), until a constant weight and full and homogeneous uniformity |
| | (equal to nimesulide | 50 mg) | |
| | Eudragit L 12.5 (dry substance) | (32 mg) | |
| | Eudragit S 12.5 (dry substance) | (15 mg) | |
| | PEG 400 | 37 mg | |
| | Triethyl citrate | 3 mg | |
| | Talc | 1 mg | |
| Coating C: protective film with mixed lattice with portion soluble at pH > 7.00 and insolube part | NSP | 250 mg | Microngranules are obtained by coating of NSP in Glatt (fluidized bed), until a constant weight and full and homogeneous uniformity |
| | (equal to nimesulide | 50 mg) | |
| | Eudragit S 12.5 P (dry substance) | (32 mg) | |
| | Eudragit RS (15 mg) (dry substance) | 12.5 | |
| | PEG 400 | 50 mg | |
| | Triethyl citrate | 3 mg | |
| | Talc | 1 mg | |
| Mixing of microgranules with coating B - C + microgranules A | | | Homogeneous mixing of microgranules obtained in Glatt with microgranules A is carried out in a normal biconic mixer (Gallo), over a period of time at least as long as 20' |
| Tablets: Base unit (pellet) designed for subsequent coating step in a rotating pan (coated) | Homogeneous mixing of microgranules A-B and C CMP | 100 mg | Components are submitted to direct compression by a Ronchi compressing machine, with formation of pellets of appropriate size and weight |
| | PVP | 20 mg | |
| | Magnesium stearate | 2.5 mg | |
| Coating process: pellets are submitted to homogeneous filming in a rotating pan | Pellets Hydroxyethylcellulose | 40 mg | Coating is carried out in a rotating pan to form pills with a spray process |
| | Triethyl citrate | 5 mg | |
| | Talc | 1 mg | |

### STUDY ON THE CONTROLLED RELEASE "IN VITRO" OF FORMULATION 2

The microgranule mixture was submitted to control of dissolution "in vitro" by the USP XXIII method modified in the composition of the simulated gastrointestinal fluids (of an exactly known volume).

The amount of nimesulide was determined by HPLC, against an external standard of known title: analyses gave the following results:
A. After 1 hour (pH 1.50): complete disgregation of the protective coating and the pellet with release of powder and microgranules in solution; the dosed-nimesulide amount is equal to 104.22 mg (first simulating liquid);
B. From the second to the third hour (pH 4.5 ÷ 6.9): no further disgregation of microgranules with release of powder in solution; the dosed-nimesulide amount is equal to 2.76 mg (second simulating liquid);
C. After 4 hours (pH 6.90): further disgregation of microgranules with release of powder in solution. The dosed-nimesulide amount is equal to 48.15 mg (third simulating liquid):
D. From the fifth to the seventh hour (pH 6.90 ÷ 7.20): no further disgregation. The dosed-nimesulide amount is equal to 1.89 mg (third simulating liquid);
E. After 8 hours (pH 7.20): a complete disgregation of the microgranules present is observed with release of powder in solution. The dosed-nimesulide amount is equal to 48.44 mg (fourth simulating liquid).

FORMULATION 3: single-dose packet of immediate release.

| **Description** | **Components** | | **Technology therein employed** |
|---|---|---|---|
| Active ingredient granules | NSP | 500 mg | Powders are sieved by a vibrating screen so as to obtain a homogeneous particle size. Microgranules are produced through wet granulation in a fluidized bed, through normal process parameters, using a granulating solution obtained by dissolving Cetomacrogol 1000 and an appropriate amount of sucrose in purified water. |
| | (equal to nimesulide | 100 mg) | |
| | Cetomacrogol | 8 mg | |
| | Sucrose | 1842 mg | |
| | Hydrated citric acid | 50 mg | |
| | Orange flavour | 50 mg | |
| Gauging | | | The particle size is made homogeneous through gauging carried out on an oscillating granulator |
| End mixing | | | Homogeneous mixing of the obtained granules in a fluidized bed is carried out through a normal mixer with a moving body (e.g. a biconic mixer) over a period of time of about 20-30 minutes |
| Packaging in packets | | | The homogeneous granules are packaged in single-dose packets, each containing 3 g, suitably heat-sealed by an appropriate machine |

The pharmaceutical preparations being the object of the present invention (example 1 and example 2), are adapted to enable a release of the active ingredient in two, three or more different periods of time and at different pH values, with a Cmax lower than 1.5 hours.

The substances utilized for mere coating for organoleptic purposes (smell and taste) are those known and widely validated by the present pharmaceutical technology, such as more or less-substituted celluloses for example, e.g. methylcellulose, hydroxypropylcellulose and hydroxyethylpropylcellulose.

Advantageously, the pharmaceutical forms being the object of the present invention can be in the form of tablets, capsules and packets, both of an immediate action and a delayed or controlled-release action, extemporary syrup, extemporary drinkable tiny bottles, creams, ointments and collyria to be used in the anti-inflammatory, antipyretic and analgesic therapy.

Development of a form characterized by a greater dissolution velocity as compared with traditional formulations by an appropriate selection of pharmaceutically acceptable excipients and/or vehicles, and an appropriate pharmaceutical technology, ensures a higher and quicker absorption of the molecule and a more marked and earlier clinical effect with a dual advantage: on the one hand, the possibility of a better use of the substance in painful situations of the acute type requiring a strong analgesic effect and of quick appearance, on the other hand a greater tolerability of nimesulide by oral administration, because it is likely that, since a pharmaceutical form of improved absorption is available, a reduced dose can be used as compared with those presently suggested as dosage.

In connection with the above, preparation of other useful pharmaceutical forms is made possible such as for example a gum gel to be used in the inflammatory forms of the mouth mucosa.

## Claims

1. An inclusion complex of a general formula [nimesulide:polymeric carrier], in which nimesulide is adsorbed on the pharmaceutically acceptable polymeric carrier, selected from the group comprising the hydrophilic polymers insoluble in water.

2. A complex as claimed in claim 1, in which the hydrophilic polymer insoluble in water is selected from: polyvinyl pyrrolidone and derivatives thereof, Polyplasdone XL, Polyplasdone XL-1 and Polyplasdone INF-10.

3. A complex as claimed in claim 1, in which the nimesulide and polymeric carrier are in a ratio by weight included between 1:10 and 10:1.

4. A complex as claimed in claim 3, in which the nimesulide and polymeric carrier are in a ratio by weight included between 1:5 and 5:1.

5. A complex as claimed in claim 1, wherein the nimesulide is adsorbed on the polyvinyl pyrrolidone polymer in a ratio by weight of 1:4.

6. A complex as claimed in claim 1, wherein the nimesulide adsorbed on the polymeric carrier is in an amorphous form and has a higher passage speed into a solution than the nimesulide in a solid crystalline form.

7. A process for preparing the inclusion complex as claimed in claim 1, comprising a step of adsorbing the nimesulide on a polymeric carrier to obtain a complex of a general formula [Nimesulide:polymeric carrier] in which the nimesulide is present in an amorphous form.

8. A process as claimed in claim 7, in which the adsorbing step is implemented by:
- preparing a solution comprising nimesulide and a polar organic solvent;
- adding a predetermined portion of the solution comprising nimesulide to a predetermined portion of the polymeric carrier;
- eliminating the employed solvent from the solution until an inclusion complex of a general formula [nimesulide:polymeric carrier] is obtained in which the nimesulide is adsorbed on the polymeric carrier.

9. A process as claimed in claim 7, wherein the adsorbing step is implemented by:
- pre-mixing a predetermined portion of powered nimesulide with a predetermined portion of powered polymeric carrier until a homogeneous nimesulide-polymeric carrier mixture is obtained;
- co-grinding the mixture comprising the nimesulide and polymeric carrier in a high-energy mill until an inclusion complex having a general formula [nimesulide:polymeric carrier] is obtained, in which the nimesulide is adsorbed on the polymeric carrier.

10. A pharmaceutical formulation comprising an inclusion complex as claimed in claim 1, in combination with at least one polymer and at least one pharmaceutically acceptable surface-active agent.

11. A formulation as claimed in claim 10, in a form adapted for oral and rectal administrations in which the active ingredient is combined with at least one gastro-resistant and entero-soluble material and at least one pharmaceutically acceptable surface-active agent.

12. A formulation as claimed in claim 10, in the form of a unitary dosage containing 10 to 500 mg of active ingredient.

13. A formulation as claimed in claim 12, which is in a form adapted for controlled release of the active ingredient.

14. A process for preparing a pharmaceutical formulation having an analgesic action, which comprises a complex as claimed in claim 1, in which said complex is mixed with at least one gastro-resistant and entero-soluble material and at least one pharmaceutically acceptable surface-active agent.

15. An inclusion complex [nimesulide:polymeric carrier] as claimed in claim 1, for use as an analgesic medicament in an anti-inflammatory, antipyretic and analgesic therapy.

16. Use of the inclusion complex [nimesulide:polymeric carrier] as claimed in claim 1, for preparation of a medicament for treatment of diseases associated with an anti-inflammatory, antipyretic and analgesic therapy.
